Europäisches Patentamt

European Patent Office

Office européen des brevets

(11) Publication number: **0 331 280**
**A1**

## EUROPEAN PATENT APPLICATION

(21) Application number: 89300616.3

(51) Int. Cl.4: **C07C 69/675 , C07C 67/08**

(22) Date of filing: 24.01.89

(30) Priority: 29.01.88 US 149739

(43) Date of publication of application:
06.09.89 Bulletin 89/36

(84) Designated Contracting States:
AT BE CH DE ES FR GB GR IT LI LU NL SE

(71) Applicant: PITMAN-MOORE, INC.
2315 Sanders Road
Northbrook Illinois 60062(US)

(72) Inventor: Eckler, Paul E.
2705 Hulman Street
Terre Haute Indiana 47803(US)

(74) Representative: Bannerman, David Gardner et al
Withers & Rogers 4 Dyer's Buildings Holborn
London, EC1N 2JT(GB)

(54) **Method for esterifying hindered carboxylic acids.**

(57) A method for esterification of hindered carboxylic acids with polyol compounds. Hindered carboxylic acid is reacted with polyol in the presence of catalyst selected from the group consisting of stannous chloride, stannic chloride, tetrabutyl titanate, zinc carbonate, stannous oxalate, stannous octoate, butyl chlorotin dihydroxide, ferric chloride, magnesium chloride, and lithium chloride.

EP 0 331 280 A1

EP 0 331 280 A1

## METHOD FOR ESTERIFYING HINDERED CARBOXYLIC ACID

## BACKGROUND OF THE INVENTION

### 1. Field of the Invention

This invention relates to a method for esterifying hindered carboxylic acids by reaction with polyols.

### 2. Description of Related Art

Use of resins in diverse commercially important applications is well-established. The ability to prepare resins which have pre-selected properties has increased acceptance of such materials. However, the physical form of such resins often makes them difficult to distribute in commerce. Polyester and alkyd resins prepared from polyols are well-known. Many such resins are highly viscous liquids or gelatinous semi-solids, neither of which is convenient to transport. Further, resins having high proportion of hydroxyl functionality and conveniently-transported physical form have not been made widely available because no method of synthesis is known or the method of preparation is costly.

Resinous compounds are useful, inter alia, in lacquers, waxes, and other coatings, as textile finishing treatments, and as binders for particulate matter. However, known resin products are difficult to prepare and handle. Further, known techniques for the esterification of hindered carboxylic acid moieties typically require use of strong acid catalysts, so the variety of such esters is limited. One such acid, paratoluenesulfonic acid, tends to cause discoloration of the resins and is corrosive to many of the materials of construction utilized in resin kettles.

Methods of esterifying unhindered carboxylic acids are known. For example, U.S. Patent 2.089,127 describes production of glycerol monolactate, which is useful as a solvent, by a transesterification reaction of glycerol with a lactate acid ester of a low boiling alcohol, U.S. Patent 2,488,303 teaches that esters of glycols and unhindered carboxylic acids are useful as lacquer adjuvants. However, these methods are not satisfactory for esterification of hindered carboxylic acid. U.S. Patent 3,741,941 describes esterification of an unhindered carboxylic acid with alcohol using para-toluenesulfonic acid catalyst. However, para-toluenesulfonic acid is unsatisfactory for the reasons described above.

Reaction of epoxides with a hindered carboxylic acid is known. For example, U.S. Patents 3,707,526 and 3,792,112 describe the uncatalyzed reaction of hindered carboxylic acids with an epoxy resin, optionally in the presence of a dibasic acid or a diol. Products of the reaction contain both ester and ether linkages. As U.S. Patent 3,404,018 discloses, the reaction of hydroxy-aliphatic carboxylic acid with epoxide results in cleavage of the O-H bond of the carboxyl moiety, rather than cleavage of the C-O bond. Thus the reaction of an epoxy with a carboxylic acid moiety is not hindered as is the reaction of polyol with hindered carboxylic acid. This patent further discloses that the predominant reaction is the addition of the epoxide groups to the carboxylic acid groups, with little or no reaction between carboxylic acid groups and hydroxy groups or between epoxide groups and hydroxy groups. These methods are unsatisfactory for esterifying hindered carboxylic acid moieties with polyol.

Known methods of esterifying a hindered carboxylic acid moiety are limited in scope and application. For example, U.S. Patent 3,441,953 discloses a method for preparing esters of equimolar portions of polyethylene glycol (PEG) or tetraethylene glycol (TEG) with dimethylolpropionic acid. The esters are precursors of triesters of dimethylolpropionic acid, which are useful as fabric treatments. The reaction conditions are severe; increasing temperature from $185°$ to $265°$ C, above the decomposition temperature of dimethylolpropionic acid at atmospheric pressure. Phosphorous acid, a strong acid, is utilized as catalyst, and vacuum may be necessary to remove unreacted constituents. However, these conditions do not consistently result in complete esterification of the carboxylic acid moiety on the hindered acids. Reaction of the dimethylolpropionic acid diester of distilled tallow fatty acids with a 20% stoichiometric excess of crotyl alcohol in the presence of phosphorous acid produced only about 50% yield of dimethylolpropionic acid-fatty acid diester-monocrotyl ester product. Thus, this method is unsatisfactory for consistent esterification of hindered carboxylic moieties.

Under certain circumstances, esters prepared by reacting appropriately functionalized esters with hydroxy- and carboxy-functional material are useful as "reactive diluents," i.e., modifiers of resin films. U.S. Patent 4,314,918 discloses that reactive diluents are prepared by reacting between about 0.25 to 4 mols of

2

glycidyl ester (epoxy- and carboxy-functional) with material having hydroxy moieties, carboxyl moieties, or a combination thereof. Suitable materials with which glycidyl ester is reacted include dimethylolpropionic acid, neopentyl glycol, and phthalic acid. The components may be reacted in an organic solvent, and butyl stannoic acid or dimethyl cocoamine can be present to catalyze the reaction.

Ungelled resins suitably cured by exposure to ionizing radiation or ultraviolet light are disclosed in U.S. Patent 4,649,082. The resins comprise esters which are the reaction product of a polyfunctional hydroxy-containing carboxylic acid, a hydroxyl-functional compound, and an unsaturated carboxylic acid. The esters may be prepared in one- or two-step processes. In the latter, it is broadly disclosed that the polyfunctional hydroxy-containing carboxylic acid(one example being dimethylolpropionic acid) can be reacted with an organic hydroxyl-functional compound, such as a diol, at a molar ratio of anywhere between about 3:1 to 0.5:1, preferably between 2:1 to 1.5:1, and at a temperature between about 150 and 220° C. Typically, an esterification catalyst, such as butyl stannoic acid, dibutyltin oxide, antimony oxide, dibutyltin dilaurate, para-toluenesulfonic acid, and methane sulphonic acid is utilized.

U.S. Patent 3,669,939 teaches that condensation polymers useful in coating compositions may be prepared by self-condensing polyhydroxymonocarboxylic acid monomer species in the absence of polycarboxyl-containing compounds. Small amount of monohydroxy- or polyhydroxy-containing monomers also may be co-condensed, but their presence is undesired. In the absence of polycarboxyl-containing compounds, the carboxyl is esterified by this method. The reaction is catalyzed by an esterification catalyst such as zinc naphthenate, methylene sulfonic acid, or dibutyltin oxide at temperatures less than 200° C.

The '939 patent teaches that, although mono-carboxyl, mono-hydroxy, and polyhydroxy compounds may be present in the reaction mixture, the proportions of these components preferably are minimized. The patent also teaches that exclusion of compounds containing only hydroxy groups from the reaction yields condensed polymers which are monofunctional with respect to the carboxyl group. This mono-carboxyl moiety of the polymer then can be esterified with a polyol to increase the hydroxyl functionality of the polymer, or can be modified to provide other functional moeities, such as unsaturation. These hydroxyl groups then can be esterified. However, the utility of products obtained by the direct co-condensation of polyhydroxymonocarboxylic acid and a polyhydroxy-containing constituent is not appreciated in the patent. The '939 patent discloses that high molecular weight self-condensed polyhydroxymonocarboxylic acids, or such compounds further esterified with a polyhydroxy compound, are useful in coating compositions.

U.S. Patent 4,622,117 teaches the use of an acid ion exchange catalyst instead of a corrosive catalyst to esterify the carboxyl group of dihydroxymonocarboxylic or dihydroxydicarboxylic acids, some of which are hindered. Because the number of acid sites per unit mass is low compared to an acid such as para-toluenesulfonic acid, the method requires large quantities of ion exchange catalyst. The method yields products which, inter alia, tend to turn colors. Further, the ion exchange resin must be removed from the resin by filtration. This process is difficult, especially for preparing a typically high viscosity resinous product. Also, typical exchange resins have a limited range of utility, in that, if the temperature exceeds about 250° F (120° C), they may melt, decompose, or otherwise lose their effectiveness.

It is an object of this invention to provide a method for complete esterification of hindered carboxylic acids with polyhydroxy-containing compounds.

It is another object of this invention to provide a method for complete esterification of hindered carboxylic acids with polyhydroxy-containing compounds without requiring the use of corrosive catalysts.

It is yet another object of the invention to provide a method of preparing synthetic polyol material which is the ester of hindered carboxylic acid and a polyhydroxy-containing constituent.

## SUMMARY OF THE INVENTION

In accordance with these and other objects, the subject invention relates to a method for esterification of a sterically hindered polyhydroxymonocarboxylic acid with a polyol at a mol ratio of acid to polyol of at least about 0.75:1 and at a temperature between about 80° C and the decomposition temperature of the acid in the presence of a catalytically effective quantity of active esterification catalyst. Reaction is continued until the acid number of the ester products is less than about 40.

## DETAILED DESCRIPTION OF THE INVENTION

This invention is based on the discovery that a hindered carboxylic acid moiety of certain polyhydroxy compounds can be completely esterified in the presence of certain selective catalysts to provide esters having a high proportion of hydroxyl functional groups. The esters can also incorporate fatty acids, monobasic and dibasic acids, and monoglycerides and analogous partial esters of fatty acids with polyols into the reaction product. It has been also discovered that the ester products of this reaction, which can be called synthetic polyols, are useful both as additives to coating compositions and as intermediates from which additional esterified compositions can be prepared.

In accordance with the method of this invention, a hindered carboxylic acid is reacted with a polyol in the presence of a cataylst. This reaction may take place in the presence of solvent. The carboxylic acid moiety is esterified with one hydroxyl moiety from the polyol. Esterification is an equilibrium reaction, and thus is affected by the concentration of water in the reactant mixture. If solvent is utilized, water of condensation is removed by azeotropic distillation with the solvent. Otherwise, the water of condensation evaporates at reaction conditions and is removed from the reactant mixture to ensure favorable reaction conditions. Synthetic polyol is separately recovered.

The carboxylic acid moiety of a polyhydroxymonocarboxylic acid is sterically hindered by, inter alia, hydroxyalkyl groups pendant from the quaternary carbon, i.e., the carbon to which the carboxyl group is attached. These hydroxyalkyl groups limit the ability of the carboxyl moiety to react chemically with polyols because they prevent reactive molecules from coming sufficiently close to react. Therefore, esterification of the groups is difficult and normally require severe reaction conditions.

Preferred hindered carboxylic acids include dimethylolpropionic acid, also known as 2,2-bis-(hydroxymethyl) propionic acid, trimethylol acetic acid, dimethylol butyric acid, dimethylol pentanoic acid, and blends thereof. Dimethylolpropionic acid is especially suitable.

Typically, selection of a hindered carboxylic acid is controlled by the desired properties of the ester to be prepared. Thus, dimethylolpropionic acid is preferred over dimethylol butyric acid and dimethylol pentanoic acid because it has a higher proportion of hydroxyl and carboxyl groups per unit mass. However, trimethylol acetic acid has an even higher ratio of hydroxyl to carboxyl moieties, and would be selected if this ratio was a desired characteristic of the product being prepared.

Suitable polyol esterification reactants are compounds which contain no reactive ester-producing moieties except hydroxy groups. Preferably the polyol has at least two hydroxyl groups per molecule. Again, the selection of the polyol component is a matter of establishing properties desired of the condensation product and selecting a polyol reactant which, in combination with the carboxylic acid component, produces those properties.

Suitable polyols include glycols having molecular weight up to about 2000, such as neopentyl glycol, trimethylpentanediol, cyclohexanedimethanol, alpha,omega-diols having between about 3 and 20 carbom atoms, such as 1,4-butanediol, 1,3-butylene glycol, ethylene glycol, propylene glycol, and 1,6-hexanediol. Polyols having more than 2 hydroxyl moieties, such as pentaerythritol, trimethylolethane, trimethylolpropane, glycerine, mannitol, sorbitol, and alpha-methyl glucoside, are also suitable, as are 1,2-diols having up to about 20 carbon atoms. Pentaerythritol, trimethylolethane, and trimethylolpropane are preferred because they have a high proportion of hydroxyl groups per unit mass. Pentaerythritol is especially preferred if hydroxyl content of the resulting ester is to be maximized. Other preferred polyols are 1,6-hexanediol, ethylene glycol, and 2,2,4-trimethylpentanediol-1,3. Blends of these polyols are also suitable.

The mol ratio of polyhydroxymonocarboxylic acid to polyol also is selected to produce desired physical characteristics of the resulting ester. Increasing the relative quantity of polyhydroxymonocarboxylic acid yields ester products incorporating the polyol component into molecules containing the self-conensation product of polyhydroxymonocarboxylic acid. The mol ratio of polyhydroxymonocarboxylic acid to polyol is at least about 0.75:1, preferably is between about 0.75:1 to 100:1, more preferably is between about 0.75:1 and 20:1, and most preferably is between about 0.9:1 and 2.5:1.

Products of preferred reactant mixtures in most preferred quantities are hard, glassy solids having glass transition temperatures above room temperature or are low-viscosity liquids. For example, the condensation product of equimolar amounts of dimethylolpropionic acid and pentaerythritol is a hard, glassy solid having a high glass transition temperature and a ring-and-ball softening point of about 108-110° C. Such hard, glassy synthetic polyols are useful as additives wherever hard resins would be appropriate, such as inks and coatings, including floorwax and hairspray. Because they impart hardness to their ester derivatives, these polyols are also valuable precursors to esters suitable for use as lubricants for jet engines, ink components, and preparation of alkyd and polyester resins.

The condensation products of the more preferred quantities of dimethylolpropionic acid and 1,6-hexanediol, ethylene glyol, and 2,2,4-trimethylpentanediol-1,3 are low-viscosity liquids. These synthetic polyols are useful as textile treatments, as reactive diluents in protective coating products, and as polyurethane molecule precursors.

Minor quantities of other reactants, typically less than 20% based on the total reaction mixture, may be incorporated into the synthetic polyol molecule. Known alkyd and polyester resin precursors are suitable. These components include dibasic acid such as phthalic anhydride, phthalic acid, isophthalic acid, terephthalic acid, maleic anhydride, succinic anhydride, adipic acid, azelaic acid, sebacic acid, dimer acid, and the like; monobasic acids, such as tall oil fatty acid, soybean fatty acid, linseed oil fatty acid, benzoic acid, p-t-butylbenzoic acid, hexanoic acid, heptanoic acid, octanoic acid, pelargonic acid, decanoic acid, 2-ethylhexanoic acid, lauric acid, rosin; fatty acids derived from other glycerine oils such as castor oil, coconut oil, sunflower oil, cotton seed oil, safflower oil, fish oil, and the like; and monoglycerides, which are obtained by the alcoholysis of glycerine triesters with polyols or the acidolysis of glycerine triesters with dibasic acids, as is well known in the alkyds industry.

An active esterification catalyst is used in the method of this invention. In accordance with the present invention, the active esterification catalyst is selected from the group consisting of stannous chloride, stannic chloride, tetrabutyl titanate, zinc carbonate, stannous oxalate, stannous octoate, Fascat 4101® (butyl chlorotin dihydroxide), ferric chloride, magnesium chloride, and lithium chloride. The amount of catalyst typically exceeds 0.01 wt. percent based on the weight of reactants, and is limited at its upper end only by the economic considerations. Preferably, between about 0.01-1.0, more preferably between about 0.05 and 0.5 wt. percent, and most preferably between about 0.08 and 0.13 wt. percent catalyst is used.

The activity of the tin-containing catalysts is believed to be related to the concentration of tin in the compound. Therefore, for a given quantity of the above-described tin-containing compounds, stannous chloride has the highest activity, stannous oxalate and octoate the lowest.

The activity of these catalysts makes it possible to esterify hindered carboxylic acids at temperatures above 80°C. The reaction temperature should remain below the decomposition temperature of the reactants. Within this range, the reaction temperature should be maximized to take advantage of the improvement in reaction rate as temperature is increased. For example, dimethylolpropionic acid decomposes at about 225°C at atmospheric pressure. Therefore, the preferred range of reaction temperatures for the esterification of dimethylolpropionic acid is 80°-220°C, more preferably between about 150°-220°C, and most preferably between about 200°-220°C. Those skilled in the art will be able to determine the appropriate corresponding temperature ranges for other hindered carboxylic acids.

The pressure which the reaction proceeds is up to about 20 atmospheres, preferably up to about 1.5 atmospheres. Pressures at the high range may be necessary to minimize loss of volatile reactants, while vacuum may be utilized to increase reaction rate by removing of water of condensation if the reactants and products have low volatility. Those skilled in the art are familiar with such adjustments.

Reaction of the hindered carboxylic acid and polyol typically is carried out under an inert atmosphere. Suitable inert atmospheric components include nitrogen and carbon dioxide. The reaction is allowed to continue until the acid number is reduced to less than about 40, preferably to less than about 20, and more preferably to less than about 10.

Acid number, also referred to as acid value, is the number of milligrams of potassium hydroxide (KOH) required to neutralize the acid moieties in one gram of ester. The value is determined by methods known to skilled practitioners. Typically, a weighted sample of ester is dissolved in a neutral solvent and is titrated with 0.1 N potassium hydroxide in methanol to a phenolphthalein end point. The neutral solvent is selected in accordance with the nature of the ester sample.

The products of the invention are recovered from the reaction vessel. Because the water of condensation is removed during reaction, only the desired product remains in the vessel. Molten resins may be filtered if desired to remove traces of insoluble material. The product then is cooled. If appropriate, the melt is applied to a chilled metal roller to prepare flaked resinous products suitable for bagging. Other methods of handling are known to those skilled in the art.

The following examples will illustrate the invention and should not be considered to limit the invention in any way. The invention is limited only by the scope of the appended claims.

## EXAMPLE 1

The method of the invention was utilized to prepare various esters. The identities of the components are provided in Table 1 below. A mixture of the components was heated to 220°C under a nitrogen atmosphere unless otherwise noted. Each of the products was an ester polyol.

## TABLE 1

| Example | Acid | | Polyol | | Catalyst | | Comment |
|---|---|---|---|---|---|---|---|
| | Name | Grams | Name | Grams | Name | Grams | |
| A<br>B | DMPrA<br>DMPrA | 268<br>268 | TMP<br>TMP | 270<br>270 | SC<br>TBT | 1.51<br>1.5 | Acid Value 9; Very slight haze |
| Key for Table 1 | | | | | | | |
| DMPrA Dimethylolpropionic Acid<br>TMP Trimethylolpropane<br>SC Stannic Chloride<br>TBT Tetrabutyl Titanate (Tyzor TBT) | | | | | | | |

Properties of these examples are summarized in Table 2 below. Ring and ball softening points were determined by ASTM method E-28. Viscosities, determined on an ICI cone and plate viscometer, are reproduced below in the format X/Y/Z, wherein X is the scale reading (0-10), Y is the range of the scale (10, 40, and 100 poise - same as the cone identity number), and Z equals temperature, $^{\circ}$C, at which the viscosity was determined.

### TABLE 2

| Example R & B, $^{\circ}$C | ICI Data | Gardner Color |
|---|---|---|
| A<br>B | 0.1/40/125<br>0.15/40/125 | (High)<br>1 |

Although preferred embodiments of this invention have been discussed herein, those skilled in the art will appreciate that changes and modifications may be made without departing from the spirit of this invention, as defined in and limited only by the scope of the appended claims.

## Claims

1. A method of esterifying sterically hindered polyhydroxymonocarboxylic acid comprising reacting sterically hindered polyhdroxymonocarboxylic acid and a polyol at a mol ratio of acid to polyol of at least about 0.75:1 and a temperature of between about 80 $^{\circ}$C and the decomposition temperature of the acid, in the presence of a catalytically effective quantity of an active esterification catalyst selected from stannous chloride, stannic chloride, tetrabutyl titanate, zinc carbonate, stannous oxalate, stannous octoate, butyl chlorotin dihydroxide, ferric chloride, magnesium chloride, and lithium chloride, to produce an ester and continuing said reaction until the acid number of the resulting ester is less than about 40.

2. The method of Claim 1 wherein said acid is selected from dimethylolpropionic acid, trimethylol acetic acid, dimethyl butyric acid, dimethyl pentanoic acid, and mixtures thereof.

3. The method of Claim 2 wherein the catalyst is selected from tetrabutyl titanate, zinc carbonate, ferric chloride, magnesium chloride, lithium chloride, stannous chloride, stannic chloride, stannous oxalate, and stannous octoate.

4. The method of Claim 2 wherein the quantity of said catalyst is up about 1.0 wt. percent based on the total mass of said acid and said polyol.

5. The method of Claim 2 wherein the mol ratio is between about 0.75:1 and 20:1.

6. The method of Claim 5 wherein the mol ratio is between about 0.9:1 and 2.5:1.

7. The method of Claim 1 wherein the acid number is less than 20.

8. The method of Claim 2 wherein the acid number is less than 10.

9. The method of Claim 2 wherein the polyol is selected from pentaerythritol, trimethylolethane, trimethylolpropane, 1,6-hexanediol, 2,2,4-trimethylpentanediol-1,3,ethyleneglycol, and blends thereof.

10. The method of Claim 5 wherein the polyol is selected from pentaerythritol, trimethylolethane, trimethylolpropane, 1,6-hexanediol, 2,2,4- trimethylpentanediol-1,3,ethyleneglycol, and blends thereof.

11. The method of Claim 2 wherein the temperature is adjusted to between about 150 and 220° C.

7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US-A-3 441 953 (TH.P. DUMONT) --- | 1 | C 07 C 69/675 |
| D,A | US-A-4 649 082 (CH.B. FRIEDLANDER) --- | 1 | C 07 C 67/08 |
| A | US-A-4 496 487 (D.E. PEERMAN) ----- | 1 | |

TECHNICAL FIELDS SEARCHED (Int. Cl.4)

C 07 C 69/00
C 07 C 67/00

The present search report has been drawn up for all claims

| Place of search | Date of completion of the search | Examiner |
|---|---|---|
| THE HAGUE | 01-05-1989 | KINZINGER J.M. |